# EUROPEAN PATENT SPECIFICATION

(11) **EP 2 131 720 B1**
(45) Date of publication and mention of the grant of the patent: **22.04.2015**
(21) Application number: 08826304.1
(22) Date of filing: 03.03.2008
(51) Int. Cl.: A61B 3/00, A61B 19/00

(54) **OPHTHALMIC ENDOILLUMINATOR WITH HYBRID LENS**
ENDOILLUMINATOR FÜR DAS AUGE MIT HYBRIDLINSE
ENDO-ILLUMINATEUR OPHTALMIQUE POURVU D'UNE LENTILLE HYBRIDE

(30) Priority: 09.04.2007 US 697819
(43) Date of publication of application: 16.12.2009
(73) Proprietor: Novartis AG, 4056 Basel (CH)
(72) Inventor: SMITH, Ronald T., Newport Coast, CA 92657 (US)
(74) Representative: Hanna, Peter William Derek
(86) International application number: PCT/US2008/055671
(87) International publication number: WO 2009/009165

(56) References cited:
- WO-A-96/13235
- WO-A-2005/016118
- US-A- 5 382 987
- US-A- 5 624 438
- US-A1- 2008 225 232
- DUBIK B ET AL: "Hybrid lens with corrected sphero-chromatic aberration" OPTICS AND LASER TECHNOLOGY, ELSEVIER SCIENCE PUBLISHERS BV., AMSTERDAM, NL, vol. 27, no. 5, 1 October 1995 (1995-10-01), pages 315-319, XP004026182 ISSN: 0030-3992
- STONE T ET AL: "HYBRID DIFFRACTIVE-REFRACTIVE LENSES AND ACHROMATS" APPLIED OPTICS, OSA, OPTICAL SOCIETY OF AMERICA, WASHINGTON, DC, vol. 27, no. 14, 1 July 1988 (1988-07-01), pages 2960-2971, XP002932475 ISSN: 0003-6935

## Description

### BACKGROUND OF THE INVENTION

The present invention relates to an illuminator for use in ophthalmic surgery and more particularly to an ophthalmic endoilluminator utilizing a hybrid aspheric lens to produce a light suitable for illuminating the inside of an eye.

Anatomically, the eye is divided into two distinct parts - the anterior segment and the posterior segment. The anterior segment includes the lens and extends from the outermost layer of the cornea (the corneal endothelium) to the posterior of the lens capsule. The posterior segment includes the portion of the eye behind the lens capsule. The posterior segment extends from the anterior hyaloid face to the retina, with which the posterior hyaloid face of the vitreous body is in direct contact. The posterior segment is much larger than the anterior segment.

The posterior segment includes the vitreous body—a clear, colorless, gel-like substance. It makes up approximately two-thirds of the eye's volume, giving it form and shape before birth. It is composed of 1% collagen and sodium hyaluronate and 99% water. The anterior boundary of the vitreous body is the anterior hyaloid face, which touches the posterior capsule of the lens, while the posterior hyaloid face forms its posterior boundary, and is in contact with the retina. The vitreous body is not free-flowing like the aqueous humor and has normal anatomic attachment sites. One of these sites is the vitreous base, which is a 3-4 mm wide band that overlies the ora serrata. The optic nerve head, macula lutea, and vascular arcade are also sites of attachment. The vitreous body's major functions are to hold the retina in place, maintain the integrity and shape of the globe, absorb shock due to movement, and to give support for the lens posteriorly. In contrast to aqueous humor, the vitreous body is not continuously replaced. The vitreous body becomes more fluid with age in a process known as syneresis. Syneresis results in shrinkage of the vitreous body, which can exert pressure or traction on its normal attachment sites. If enough traction is applied, the vitreous body may pull itself from its retinal attachment and create a retinal tear or hole.

Various surgical procedures, called vitreo-retinal procedures, are commonly performed in the posterior segment of the eye. Vitreo-retinal procedures are appropriate to treat many serious conditions of the posterior segment. Vitreo-retinal procedures treat conditions such as age-related macular degeneration (AMD), diabetic retinopathy and diabetic vitreous hemorrhage, macular hole, retinal detachment, epiretinal membrane, CMV retinitis, and many other ophthalmic conditions.

A surgeon performs vitreo-retinal procedures with a microscope and special lenses designed to provide a clear image of the posterior segment. Several tiny incisions just a millimeter or so in length are made on the sclera at the pars plana. The surgeon inserts microsurgical instruments through the incisions such as a fiber optic light source to illuminate inside the eye, an infusion line to maintain the eye's shape during surgery, and instruments to cut and remove the vitreous body.

During such surgical procedures, proper illumination of the inside of the eye is important. Typically, a thin optical fiber is inserted into the eye to provide the illumination. A light source, such as a metal halide lamp, a halogen lamp, a xenon lamp, or a mercury vapor lamp, is often used to produce the light carried by the optical fiber into the eye. The light passes through several optical elements (typically lenses, mirrors, and attenuators) and is launched at the optical fiber that carries the light into the eye. The quality of this light is dependent on several factors including the types of optical elements selected.

As is commonly known, the refractive surfaces of lenses produce chromatic aberrations. The exit angle of a light ray passing through a refractive surface is negatively correlated to wavelength - the higher the wavelength of the light, the lesser the resulting exit angle relative to the incident angle. In this manner, lenses used in traditional ophthalmic endoilluminators produce chromatic aberrations which result in color and brightness non-uniformities in the light emitted into the eye. In addition, these aberrations also slightly reduce the luminous flux of an output beam and increase the beam diameter - both of which potentially reduce the quality of the light emitted into the eye from the optical fiber. What is needed is an improved ophthalmic endoilluminator that minimizes the non-uniformities associated with traditional refractive lenses.

The state of the art is represented by WO-A-2005/016118 (Auld, et al) and Dubik B. et al, "Hybrid lens with corrected sphero-chromatic aberration" OPTICS AND LASER TECHNOLOGY, Elsevier Science Publishers BV, Amsterdam, NL, vol. 27, no. 5, 1st October 1995, pages 315-319, ISSN: 0030-3992.

### SUMMARY OF THE INVENTION

The present invention provides an ophthalmic endoilluminator in accordance with claims which follow.

In one embodiment consistent with the principles of the present invention, the present invention is an ophthalmic endoilluminator having a light source, a collimating lens for collimating the light produced by the light source, a hybrid condensing lens for focusing the light, and an optical fiber for carrying the focused light into an eye.

In another embodiment consistent with the principles of the present invention, the present invention is an ophthalmic endoilluminator having a light source, a hybrid collimating lens for collimating the light produced by the light source, a condensing lens for focusing the light, and an optical fiber for carrying the focused light into an eye.

The hybrid lens has a refractive surface for refracting a light beam and a diffractive surface for diffracting the light beam. The diffractive surface is designed to compensate for chromatic aberration caused by the refractive surface.

It is to be understood that both the foregoing general description and the following detailed description are exemplary and explanatory only and are intended to provide further explanation of the invention as claimed. The following description, as well as the practice of the invention, set forth and suggest additional advantages and purposes of the invention.

### BRIEF DESCRIPTION OF THE DRAWINGS

The accompanying drawings, which are incorporated in and constitute a part of this specification, illustrate several embodiments of the invention and together with the description, serve to explain the principles of the invention.
Figure 1 is an unfolded view of an ophthalmic endoilluminator according to an embodiment of the present invention.
Figure 2A is a view of the light ray pattern of a traditional condensing lens.
Figures 2B-2F are exploded views of different light ray patterns of a traditional condensing lens.
Figures 3A-3C depict a condensing lens according to an embodiment of the present invention.
Figure 4 is a view of the light ray pattern of a conventional collimating lens.
Figures 5A-5B depict a collimating lens according to an embodiment of the present invention.
Figure 6 is a partial view of a different ophthalmic endoilluminator.
Figure 7 is a partial view of an ophthalmic endoilluminator according to an embodiment of the present invention.
Figure 8 is a view of an endoilluminator probe as used in an eye according to an embodiment of the present invention.

### DETAILED DESCRIPTION OF THE PREFERRED EMBODIMENTS

Reference is now made in detail to the exemplary embodiments of the invention, examples of which are illustrated in the accompanying drawings. Wherever possible, the same reference numbers are used throughout the drawings to refer to the same or like parts.

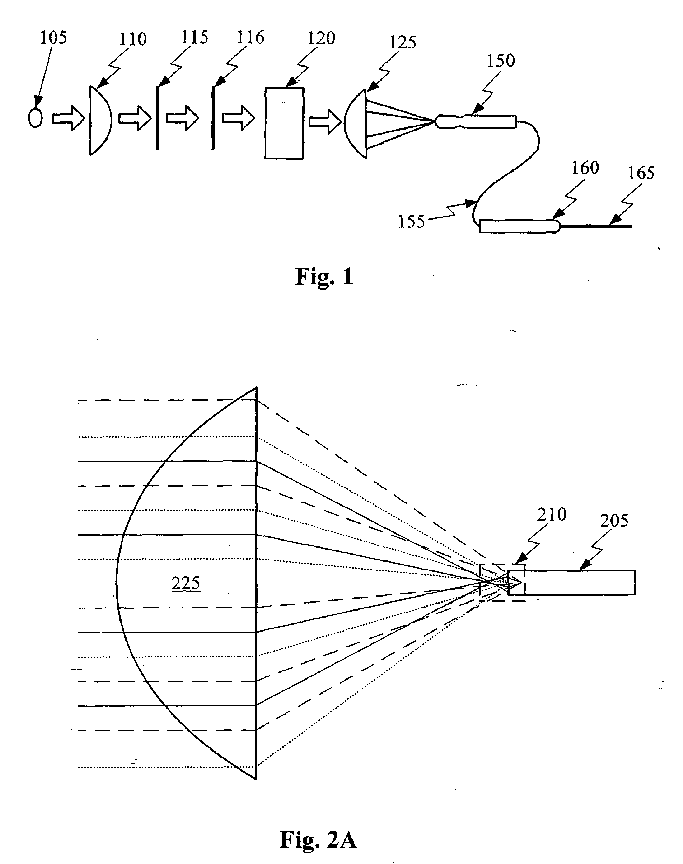

Figure 1 is an unfolded view of an ophthalmic endoilluminator according to an embodiment of the present invention. In Figure 1, the endoilluminator includes light source 105, collimating lens 110, optional cold mirror 115, optional hot mirror 116, attenuator 120, condensing lens 125, connector 150, optical fiber 155, hand piece 160, and probe 165.

The light from light source 105 is collimated by collimating lens 110. The collimated light is reflected and filtered by optional cold mirror 115 and/or transmitted and filtered by optional hot mirror 116. The resulting beam is attenuated by attenuator 120 and focused by condensing lens 125. The focused beam is directed through connector 150 and optical fiber 155 to probe 165 where it illuminates the inside of the eye.

Light source 105 is typically a lamp, such as a mercury vapor lamp, a xenon lamp, a metal halide lamp, or a halogen lamp. Light source 105 is operated at or near full power to produce a relatively stable and constant light output. In one embodiment of the present invention, light source 105 is a xenon lamp with an arc length of about 0.18 mm. Other embodiments of the present invention utilize other light sources such as light emitting diodes (LEDs). One or more LEDs can be operated to produce a constant and stable light output. As is known, there are many types of LEDs with different power ratings and light output that can be selected as light source 105.

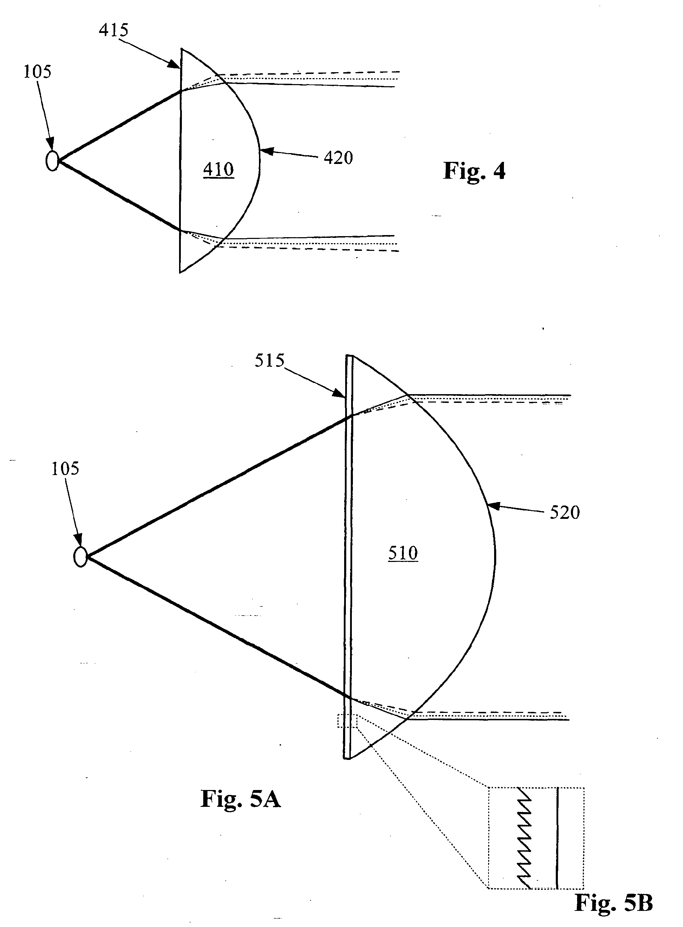

Collimating lens 110 is configured to collimate the light produced by light source 105. As is commonly known, collimation of light involves lining up light rays. Collimated light is light whose rays are parallel with a planar wave front. In one embodiment of the present invention, collimating lens 110 is a hybrid lens as described in Figure 4.

Optional cold mirror 115 is a dichroic reflector that reflects visible wavelength light and only transmits infrared and ultraviolet light to produce a beam filtered of harmful infrared and ultraviolet rays. Optional hot mirror 116 reflects long wavelength infrared light and short wavelength ultraviolet light while transmitting visible light. The eye's natural lens filters the light that enters the eye. In particular, the natural lens absorbs blue and ultraviolet light which can damage the retina. Providing light of the proper range of visible light wavelengths while filtering out harmful short and long wavelengths can greatly reduce the risk of damage to the retina through aphakic hazard, blue light photochemical retinal damage and infrared heating damage, and similar light toxicity hazards. Typically, a light in the range of about 430 to 700 nanometers is preferable for reducing the risks of these hazards. Optional cold mirror 115 and optional hot mirror 116 are selected to allow light of a suitable wavelength to be emitted into an eye. Other filters and/or dichroic beam splitters may also be employed to produce a light in this suitable wavelength range. For example, holographic mirrors may also be used to filter light.

Attenuator 120 attenuates or decreases the intensity of the light beam. Any number of different attenuators may be used. For example, mechanical louvers, camera variable aperture mechanisms, or neutral density filters may be used. A variable-wedge rotating disk attenuators may also be used.

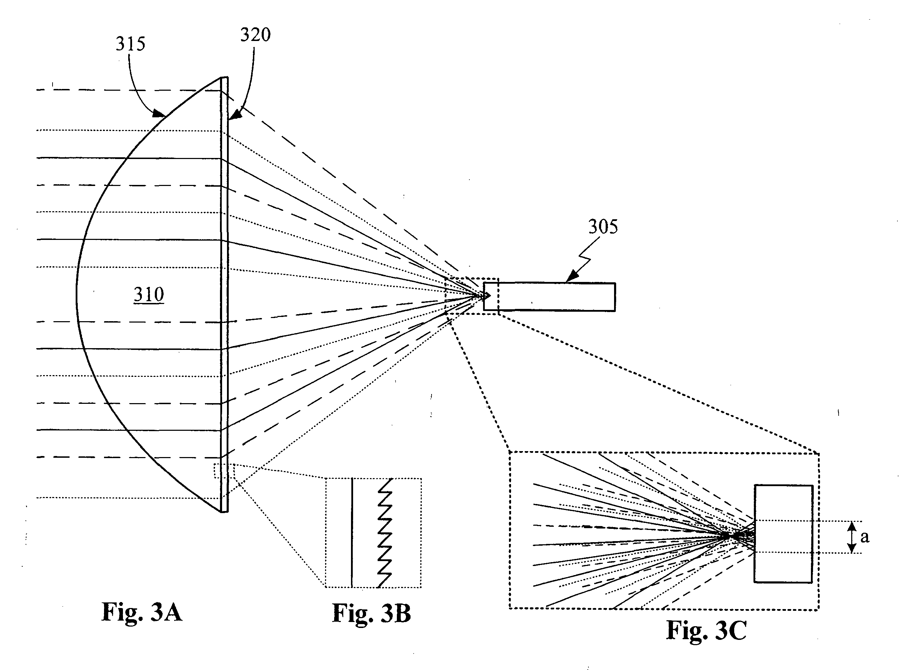

Condensing lens 125 focuses the attenuated light beam so that it can be launched into a small diameter optical fiber. Condensing lens 125 is a lens of suitable configuration for the system. Condensing lens 125 is typically designed so that the resulting focused beam of light can be suitably launched into and transmitted by an optical fiber. As is commonly known, a condensing lens may be a biconvex or plano-convex spherical or aspheric lens. In a plano-convex aspheric lens, one surface is planar and the other surface is convex with a precise aspheric surface in order to focus the light to a minimum diameter spot. In one embodiment of the present invention, condensing lens 125 is a hybrid lens as described in Figure 3.

The endoilluminator that is handled by the ophthalmic surgeon includes connector 150, optical fiber 155, hand piece 160, and probe 165. Connector 150 is designed to connect the optical fiber 155 to a main console (not shown) containing light source 105. Connector 150 properly aligns optical fiber 155 with the beam of light that is to be transmitted into the eye. Optical fiber 155 is typically a small diameter fiber that may or may not be tapered. Hand piece 160 is held by the surgeon and allows for the manipulation of probe 165 in the eye. Probe 165 is inserted into the eye and carries optical fiber 155 which terminates at the end of probe 165. Probe 165 thus provides illumination from optical fiber 155 in the eye.

Figure 2A is a view of the light ray pattern of a traditional collimating lens/ condensing lens pair. In Figure 2A, condensing lens 225 is a refractive aspheric lens, such as a commonly available plano-convex refractive aspheric lens. The angle of the light rays refracted by condensing lens 225 is a function of the wavelength of those light rays. A larger wavelength yields a smaller bend angle. In this manner, red light has a smaller bend angle than green light, which in turn has a smaller bend angle than blue light. This results in chromatic aberration in the light beam carried by optical fiber 205. In Figures 2A-2F, red light is depicted by the dashed lines, green light by the dotted lines, and blue light by the solid lines.

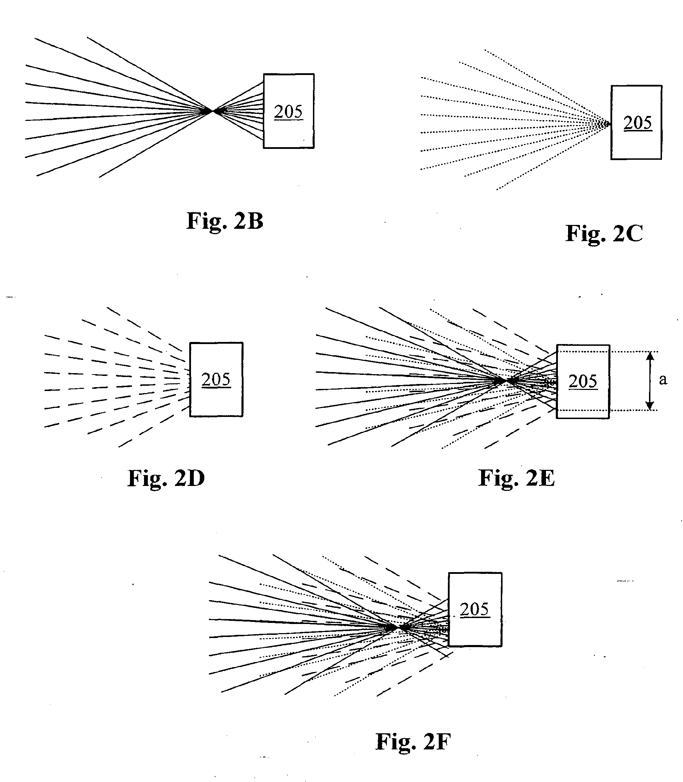

Condensing lens 225 launches a light beam at optical fiber 205. In Figure 2A, light rays are depicted by the lines that converge in box 210. Figures 2B-2F are exploded views of different light ray patterns of a traditional condensing lens as depicted in box 210 of Figure 2A. Figure 2B depicts blue light; Figure 2C depicts green light; Figure 2D depicts red light; Figure 2E depicts a combination of blue, green, and red light; and Figure 2F depicts a combination of blue, green, and red light in which optical fiber 205 is misaligned with the light beam.

Figure 2B depicts blue light rays that have been refracted by condensing lens 225. Since blue light has a wavelength that is smaller than that of green and red light, the bend angle is greater than that of green and red light. In Figure 2B, the blue light rays converge at a point on the condensing lens side of the optical fiber 205. In such a manner, the blue light rays begin to diverge before striking the end of optical fiber 205.

Figure 2C depicts green light rays that have been refracted by condensing lens 225. Since green light has a wavelength that is smaller than that of red light and greater than that of blue light, the bend angle is between that of green and red light. In other words, the bend angle of green light refracted by condensing lens 225 is intermediate. In Figure 2C, the green light rays converge at a point very near the surface of the optical fiber 205. In such a manner, the green light rays are focused at the end of optical fiber 205.

Figure 2D depicts red light rays that have been refracted by condensing lens 225. Since red light has a wavelength that is greater than that of green and blue light, the bend angle is smaller than that of green and blue light. In Figure 2C, the red light rays converge at a point within the optical fiber 205. In such a manner, the red light rays have not converged before striking the end of optical fiber 205.

Figure 2E depicts a composite light beam made up of the blue, green, and red light rays depicted in Figures 2B-2D. The face of optical fiber 205 receives the combination beam that has a beam diameter of "a." In this case, the beam diameter "a" is relatively large - approximately 657 microns for a standard illuminator using a high luminance xenon arc lamp. This requires a fiber with a large proximal diameter in order to ensure high coupling efficiency of light into the fiber. Furthermore, transmittance vs. incident angle in an optical fiber is generally a complex function of spatial position of the light at the fiber proximal entrance aperture. In the focused light pattern of Figure 2E it is apparent that the distribution of ray angles vs. spatial position at the fiber entrance aperture is strongly dependent on wavelength. As a result, the transmittance vs. exit angle from the fiber is also wavelength dependent. This can result in color and brightness angular non-uniformities in the light emitted from the distal end of the optical fiber (the light emitted into an eye).

Another problem associated with a relatively large beam diameter is depicted in Figure 2F in which the light beam launched at the tapered optical fiber 205 is laterally misaligned with that fiber. In such a case, not all of the light content of the light beam is carried by the optical fiber. Furthermore, it is apparent from Figure 2F that the portion of light that gets spatially clipped by the fiber entrance aperture is dependent on wavelength and angle. This further contributes to the color and brightness angular non-uniformities in the light emitted from the distal end of the optical fiber (the light emitted into an eye).

Figures 3A-3C depict a condensing lens according to an embodiment of the present invention. Hybrid condensing lens 310 has both refractive and diffractive surfaces (hybrid refractive / diffractive aspheric lens). Hybrid condensing lens 310 has a refractive aspheric convex surface 315 and a diffractive planar surface 320. The structure of the diffractive surface is shown more clearly in Figure 3B. In Figure 3B, the diffractive surface consists of small teeth-like facets similar to but much smaller than those found on a fresnel lens. This diffractive surface is also like a blazed grating on the planar surface 320 of hybrid condensing lens 310. In other embodiments consistent with the principles of the present invention, the refractive condensing lens 310 and diffractive planar surface may be implemented in two separate components.

Hybrid condensing lens 310 both refracts and diffracts the light beam that passes through it. When the diffractive surface 320 is illuminated with a converging white light beam, the diffractive surface diffracts the beam towards a focus slightly closer to the diffractive surface than the incident beam. The diffractive surface 320 also exhibits chromatic dispersion in which longer wavelength light is bent the most. The chromatic dispersion from the diffractive surface 320 is in the opposite direction of the chromatic aberration of the aspheric refractive lens 315. A diffractive surface 320 can be designed to diffract nearly 100% of the incident white light into the main diffracted (i.e. +1 order) direction. In other words, the chromatic aberration produced by the refractive surface 315 can be substantially canceled by the chromatic dispersion of the diffractive surface 320.

The resulting light beam produced by hybrid condensing lens 310 is shown in Figure 3C. As shown, the diameter "a" of the resulting light beam is relatively small - approximately 103 microns as compared to approximately 657 microns in Figures 2A-2F. This more focused light beam allows for design choices that include more compact optics and a smaller optical fiber 305. In addition, the light beam exhibits reduced color and brightness non-uniformities, a more spectrally flat fiber output, and a minor increase in luminous flux.

Figure 4 a view of the light ray pattern of a conventional collimating lens. In Figure 4, red light is depicted by the dashed lines, green light is depicted by the dotted lines, and blue light is depicted by the solid lines. Light source 105 is shown emitting white light (two thick, solid lines). As shown, the white light emitted by light source is refracted by first refractive surface 415 of conventional collimating lens 410. As the ray of white light passes first refractive surface 415, it is split into a typical color pattern denoted by the red, green, and blue rays of light. The light is further refracted by second refractive surface 420 of conventional collimating lens 410. As previously noted, a larger wavelength yields a smaller bend angle. In this manner, red light has a smaller bend angle than green light, which in turn has a smaller bend angle than blue light. This results in chromatic aberration depicted in Figure 4. In Figure 4, the resulting beam of red light has a larger diameter that the resulting beam of green light which in turn has a larger diameter than the resulting beam of blue light. In addition, the red beam diverges from the center of the conventional collimating lens, and the blue beam converges toward the center of the conventional collimating lens.

Figures 5A and 5B depict a collimating lens according to another embodiment of the present invention. In Figure 5A, red light is depicted by the dashed lines, green light is depicted by the dotted lines, and blue light is depicted by the solid lines. Light source 105 is shown emitting white light (two thick, solid lines). Like hybrid condensing lens 310 of Figure 3, hybrid collimating lens 510 has both refractive and diffractive surfaces. Hybrid collimating lens 510 has a diffractive planar surface 515 and a refractive aspheric convex surface 520. The structure of the diffractive surface is shown more clearly in Figure 5B. In Figure 5B, the diffractive surface consists of small teeth-like facets similar to but much smaller than those found on a fresnel lens. This diffractive surface is also like a blazed grating on the planar surface 515 of hybrid collimating lens 510. In a variant deviating from the present invention, the refractive collimating lens 510 and diffractive planar surface may be implemented in two separate components.

Hybrid collimating lens 510 functions in a manner similar to that of hybrid condensing lens 310 of Figure 3A. Light from light source 105 strikes diffractive surface 515 of hybrid collimating lens 510. Diffractive surface 515 produces chromatic dispersion of the light from light source 105. Refractive surface 520 produces chromatic aberration of the same light. The chromatic dispersion of the diffractive surface 515 can be substantially canceled by the chromatic aberration produced by refractive surface 520.

In Figure 5A, the hybrid collimating lens 510 compensates for the differing diameters of the different colored beams of light. As shown, the width of the blue beam is greater than the width of the green beam which in turn is greater than the width of the red beam. In this manner, using hybrid collimating lens 510 compensates for the beam diameter problem caused by a conventional collimating lens. In Figure 4, the beam exiting the collimating lens 410 has different beam diameters for the different colored beams of light. The width of the red beam is the greatest, the width of the green beam is intermediate, and the width of the blue beam is narrowest. Furthermore, as the beam propagates away from the collimating lens, the converging blue beam diameter decreases, the collimated green beam diameter remains fixed, and the diverging beam diameter increases. Therefore, the disparity in beam diameters between the blue, green and red beams increases as the distance from the collimating lens 410 increases. By the time the beam reaches condensing lens 310, in many cases the disparity in beam diameter is large. This can result in angular color and brightness nonuniformities in the beam emitted from optical fiber probe 165.

In Figure 5A, the beam exiting the hybrid collimating lens 510 has different beam diameters for the different colored beams of light in a manner opposite to that of collimating lens 410. The width of the blue beam is the greatest, the width of the green beam is intermediate, and the width of the red beam is narrowest. The beam diameter disparity at the output surface of the hybrid collimating lens 510 is similar in magnitude but opposite in direction from the beam diameter disparity at the output surface of the collimating lens 410. However, the beam exiting hybrid collimating lens 510 is collimated for all colors of light. Therefore, the beam diameter disparity with color does not increase as the distance from the hybrid collimating lens increases. Therefore, the beam diameter color disparity at condensing lens 310 of the beam exiting hybrid collimating lens 510 is much smaller than the beam diameter color disparity at condensing lens 310 of the beam exiting collimating lens 410. This results in a potential increase in angular color and brightness uniformity of the beam emitted from optical fiber probe 165.

The diffractive grating can be selected to provide any type of compensation. For example, when a hybrid collimating lens is used in conjunction with a conventional condensing lens, the diffractive grating on the hybrid collimating lens can be designed to compensate for the refractive surface on the hybrid collimating lens and the two refractive surfaces on the condensing lens. When a hybrid collimating lens is used in conjunction with a hybrid condensing lens, the diffractive grating on the hybrid collimating lens can be designed to compensate for the refractive surface on the hybrid collimating lens and the refractive surface on the condensing lens. This is the case shown in Figure 5A.

While the hybrid lenses of Figures 3 and 5 are depicted as aspheric, they may be of any suitable configuration. Likewise, while the diffractive surfaces 315 and 515 are depicted as planar, they may be non-planar or aspheric. Different diffractive surfaces 315, 515 can be utilized with different refractive surfaces to decrease chromatic aberration and other angular nonuniformities.

Moreover, the collimating lens 110 may be a hybrid lens while the condensing lens 125 may be a refractive lens. In another embodiment of the present invention, the collimating lens 110 may be a refractive lens while the condensing lens 125 may be a hybrid lens. In a variant diverging from the present invention, both the collimating lens 110 and the condensing lens 125 are hybrid lenses.

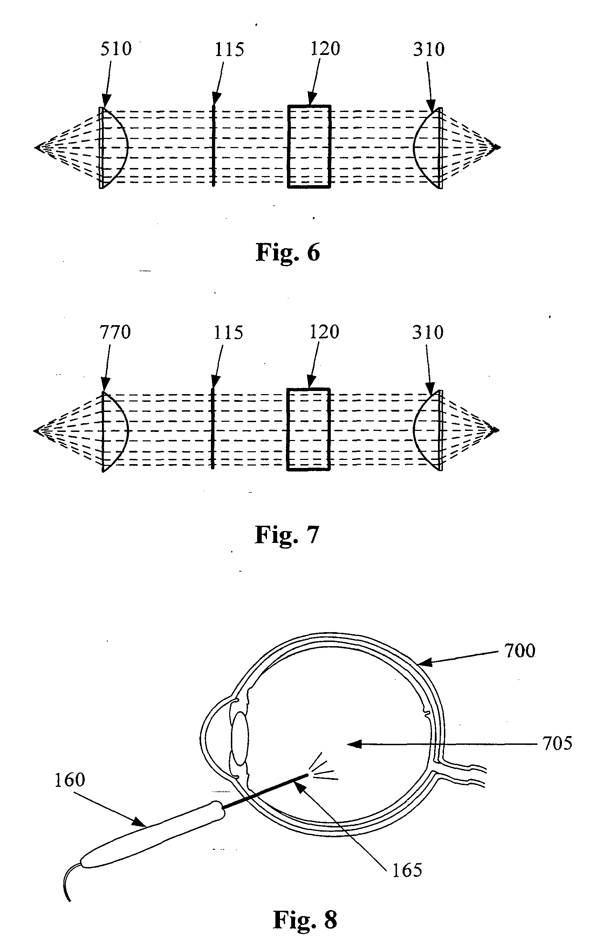

Figure 6 is a partial view of a different ophthalmic endoilluminator. In Figure 6, both collimating lens 405 and condensing lens 310 are hybrid lenses as described in Figures 3 and 4. A filter, such as cold mirror 115, and an attenuator 120 are also depicted. Light rays are depicted by the dashed lines. Hybrid condensing lens 310 is preferably made of plastic which can be easily molded to form diffractive surface 320. Because hybrid collimating lens 405 is located close to the light source, it is preferably made of glass which can better withstand heat. Hybrid collimating lens 405 collimates the light beam with a minimum of chromatic aberration and hybrid condensing lens 310 focuses the collimated light beam with a minimum of chromatic aberration.

Figure 7 is a partial view of an ophthalmic endoilluminator according to another embodiment of the present invention. In Figure 7, collimating lens 770 is a standard refractive lens, and condensing lens 310 is a hybrid lens as described in Figures 3A-3C. A filter, such as cold mirror 115, and an attenuator 120 are also depicted. Light rays are depicted by the dashed lines. Hybrid condensing lens 310 is preferably made of plastic which can be easily molded to form diffractive surface 320. Hybrid condensing lens can be designed to compensate for the other three refractive surfaces - two refractive surfaces on collimating lens 770 and one refractive surface on hybrid condensing lens 310. The result is a tightly focused light beam with greatly reduced color and brightness non-uniformities and slightly increased luminous flux. In one embodiment consistent with the principles of the present invention and which uses a high luminance xenon arc lamp, the beam's focused diameter is approximately 100 microns, its CIE 1931 (x,y) chromaticity is approximately (0.316, 0.354), and its aphakic sum normalized to 10 lumens is about 6.37 milliwatts.

Figure 8 is cross section view of an ophthalmic endoilluminator located in an eye according to an embodiment of the present invention. Figure 8 depicts hand piece 160 and probe 165 in use. Probe 165 is inserted into eye 700 through an incision in the pars plana region. Probe 165 illuminates the inside or vitreous region 705 of eye 700. In this configuration, probe 165 can be used to illuminate the inside or vitreous region 705 of eye 700 during vitreo-retinal surgery.

From the above, it may be appreciated that the present invention provides an improved system for illuminating the inside of the eye. Utilizing a hybrid lens in an improved ophthalmic endoilluminator greatly reduces color and brightness angular non-uniformity in the light beam emitted into the eye. The diffractive surface on the hybrid lens compensates for the other refractive surfaces thus reducing color and brightness angular nonuniformities.

It is intended that the description and examples be considered as exemplary only, with the true scope of the invention being indicated by the claims.

## Claims

1. An ophthalmic endoilluminator comprising:
a light source (105) for producing light;
a collimating lens (510) for collimating the light produced by the light source, having a refractive surface (520);
a condensing lens (310) for focusing the light, having a refractive surface (315); and
an optical fiber (155) for carrying the focused light into an eye,
**characterized in that** one of the collimating lens (510) and the condensing lens (310) is a hybrid lens, having a diffractive surface and a refractive surface;
wherein when the collimating lens is the hybrid lens, its diffractive surface is adapted to substantially compensate for the chromatic aberrations caused by its own refractive surface and by the condensing lens (310);
wherein when the condensing lens is the hybrid lens, its diffractive surface is adapted to substantially compensate for the chromatic aberrations caused by its own refractive surface and by the collimating lens (510).

2. The endoilluminator of claim 1 further comprising:
an attenuator (120) for attenuating the light.

3. The endoilluminator of claim 1 further comprising:
a filter for filtering the light.

4. The endoilluminator of claim 3 wherein the filter comprises a cold mirror (115).

5. The endoilluminator of claim 3 wherein the filter comprises a hot mirror (116).

6. The endoilluminator of claim 1 wherein the diffractive surface is non-planar.

7. The endoilluminator of claim 1 wherein the hybrid lens (310) has an aspheric convex refractive surface (315) and a planar diffractive surface (320).

8. The endoilluminator of claim 1 wherein, when the hybrid lens (310) is a condensing lens, it is made of a plastic material.

9. The endoilluminator of claim 1 further comprising:
a connector (150) for aligning the attenuated light with the optical fiber (155);
a hand piece (160) carrying the optical fiber, the hand piece capable of being manipulated in the hand; and
a probe (165) for carrying the optical fiber into the eye.

10. The endoilluminator of claim 1 wherein a light beam focused by the hybrid lens, when the hybrid lens (310) is a condensing lens, has a beam diameter of about 100 microns.

11. The endoilluminator of claim 10 wherein the light beam focused by the hybrid lens, when the hybrid lens (310) is a condensing lens, has an (x,y) chromaticity of about (0.316, 0.354).

12. The endoilluminator of claim 10 wherein the light beam focused by the hybrid lens, when the hybrid lens (310) is a condensing lens, has an aphakic sum normalized to 10 lumens of about 6.37 milliwatts.

## Patentansprüche

1. Augen-Endoilluminator, aufweisend:
eine Lichtquelle (105) zum Erzeugen von Licht;
eine Kollimator-Linse (510) zum Kollimieren des durch die Lichtquelle erzeugten Lichtes, wobei die Linse eine refraktive Fläche (520) hat;
eine Kondensatorlinse (310) zum Fokussieren des Lichtes, wobei die Linse eine refraktive Fläche (315) hat; und
eine optische Faser (155) zum Tragen des fokussierten Lichtes in ein Auge,
**dadurch gekennzeichnet, dass** entweder die Kollimator-Linse (510) oder die Kondensator-Linse (310) eine Hybrid-Linse mit einer diffraktiven Fläche und einer refraktiven Fläche ist;
wobei, wenn die Kollimator-Linse die Hybrid-Linse ist, deren diffraktive Fläche dazu adaptiert ist, im Wesentlichen die chromatischen Aberrationen zu kompensieren, die durch ihre eigene refraktive Fläche und durch die Kondensator-Linse (310) entstehen;
und wobei, wenn die Kondensator-Linse die Hybrid-Linse ist, deren diffraktive Fläche dazu adaptiert ist, im Wesentlichen die chromatischen Aberrationen zu kompensieren, die durch ihre eigene refraktive Fläche und durch die Kollimator-Linse (510) entstehen.

2. Endoilluminator nach Anspruch 1, ferner aufweisend:
ein Dämpfungselement (120) zum Dämpfen des Lichts.

3. Endoilluminator nach Anspruch 1, ferner aufweisend:
einen Filter zum Filtern des Lichts.

4. Endoilluminator nach Anspruch 3, wobei der Filter einen Kaltspiegel (115) aufweist.

5. Endoilluminator nach Anspruch 3, wobei der Filter einen Wärmespiegel (116) aufweist.

6. Endoilluminator nach Anspruch 1, wobei die diffraktive Fläche nicht planar ist.

7. Endoilluminator nach Anspruch 1, wobei die Hybrid-Linse (310) eine asphärische, konvexe, refraktive Fläche (315) und eine planare diffraktive Fläche (320) hat.

8. Endoilluminator nach Anspruch 1, wobei, wenn die Hybrid-Linse (310) eine Kondensator-Linse ist, diese aus einem Kunststoffmaterial gefertigt ist.

9. Endoilluminator nach Anspruch 1, ferner aufweisend:
einen Connector (150) zum Ausrichten des gedämpften Lichtes mit einer optischen Faser (155);
ein Handstück (160), das die optische Faser trägt, wobei das Handstück in der Hand handhabbar ist; und
eine Sonde (165) zum Tragen der optischen Faser in das Auge.

10. Endoilluminator nach Anspruch 1, wobei ein durch die Hybrid-Linse fokussierter Lichtstrahl einen Strahldurchmesser von ca. 100 Mikrometer hat, wenn die Hybrid-Linse (310) eine Kondensator-Linse ist.

11. Endoilluminator nach Anspruch 10, wobei der durch die Hybrid-Linse fokussierte Lichtstrahl eine (x, y) Chromatizität von ca. (0.316, 0.354) hat, wenn die Hybrid-Linse (310) eine Kondensator-Linse ist.

12. Endoilluminator nach Anspruch 10, wobei der durch die Hybrid-Linse fokussierte Lichtstrahl eine Aphakie-Summe normiert auf 10 Lumen von ca. 6,37 Milliwatt hat, wenn die Hybrid-Linse (310) eine Kondensator-Linse ist.

## Revendications

1. Dispositif d'éclairage endoscopique ophtalmique comprenant :
une source de lumière (105) destinée à produire une lumière ;
une lentille de collimation (510) destinée à collimater la lumière produite par la source de lumière, qui présente une surface de réfraction (520) ;
une lentille de condensation (310) destinée à focaliser la lumière, qui présente une surface de réfraction (315) ; et
une fibre optique (155) destinée à acheminer la lumière focalisée dans un oeil ;
**caractérisé en ce que** l'une de la lentille de collimation (510) et de la lentille de condensation (310) est une lentille hybride, qui présente une surface de diffraction et une surface de réfraction ;
dans lequel, lorsque la lentille de collimation est la lentille hybride, sa surface de diffraction est adaptée de façon à compenser sensiblement les aberrations chromatiques provoquées par sa propre surface de réfraction et par la lentille de condensation (310) ;
dans lequel, lorsque la lentille de condensation est la lentille hybride, sa surface de diffraction est adaptée de façon à compenser sensiblement les aberrations chromatiques provoquées par sa propre surface de réfraction et par la lentille de collimation (510).

2. Dispositif d'éclairage endoscopique selon la revendication 1, comprenant en outre :
un atténuateur (120) destiné à atténuer la lumière.

3. Dispositif d'éclairage endoscopique selon la revendication 1, comprenant en outre :
un filtre destiné à filtrer la lumière.

4. Dispositif d'éclairage endoscopique selon la revendication 3, dans lequel le filtre comprend un miroir froid (115).

5. Dispositif d'éclairage endoscopique selon la revendication 3, dans lequel le filtre comprend un miroir chaud (116).

6. Dispositif d'éclairage endoscopique selon la revendication 1, dans lequel la surface de diffraction n'est pas plane.

7. Dispositif d'éclairage endoscopique selon la revendication 1, dans lequel la lentille hybride (310) présente une surface de réfraction convexe asphérique (315) et une surface de diffraction plane (320).

8. Dispositif d'éclairage endoscopique selon la revendication 1, dans lequel, lorsque la lentille hybride (310) est une lentille de condensation, elle est réalisée dans un matériau de matière plastique.

9. Dispositif d'éclairage endoscopique selon la revendication 1, comprenant en outre :
un connecteur (150) destiné à aligner la lumière atténuée avec la fibre optique (155) ;
une pièce à main (160) qui porte la fibre optique, la pièce à main pouvant être manipulée à la main ; et
une sonde (165) destinée à porter la fibre optique dans l'oeil.

10. Dispositif d'éclairage endoscopique selon la revendication 1, dans lequel un faisceau de lumière focalisé par la lentille hybride, lorsque la lentille hybride (310) est une lentille de condensation, présente un diamètre de faisceau approximativement égal à 100 micromètres.

11. Dispositif d'éclairage endoscopique selon la revendication 10, dans lequel le faisceau de lumière focalisé par la lentille hybride, lorsque la lentille hybride (310) est une lentille de condensation, présente une chromaticité (x, y) approximativement égale à (0,316, 0,354).

12. Dispositif d'éclairage endoscopique selon la revendication 10, dans lequel le faisceau de lumière focalisé par la lentille hybride, lorsque la lentille hybride (310) est une lentille de condensation, présente une somme aphaque normalisée à 10 lumens, d'approximativement 6,37 milliwatts.
